(19) Europäisches Patentamt
European Patent Office
Office européen des brevets

(11) EP 4 338 832 A1

# (12) EUROPEAN PATENT APPLICATION

published in accordance with Art. 153(4) EPC

(43) Date of publication:
**20.03.2024 Bulletin 2024/12**

(21) Application number: **22807425.8**

(22) Date of filing: **09.05.2022**

(51) International Patent Classification (IPC):
***B01J 20/26*** *(2006.01)* ***B01J 20/28*** *(2006.01)*
***B01J 20/30*** *(2006.01)* ***C08F 2/18*** *(2006.01)*
***C08F 20/06*** *(2006.01)* ***C08J 3/12*** *(2006.01)*

(52) Cooperative Patent Classification (CPC):
**B01J 20/26; B01J 20/28; B01J 20/30; C08F 2/18; C08F 20/06; C08J 3/12**

(86) International application number:
**PCT/JP2022/019629**

(87) International publication number:
**WO 2022/239723 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.05.2021 JP 2021081231**
**21.12.2021 JP 2021207381**

(71) Applicant: **Nippon Shokubai Co., Ltd.**
**Osaka-shi, Osaka 541-0043 (JP)**

(72) Inventors:
- **TSURU, Kanako**
  **Himeji-shi, Hyogo 671-1282 (JP)**
- **MATSUMOTO, Satoshi**
  **Himeji-shi, Hyogo 671-1282 (JP)**
- **KITABATA, Sachie**
  **Himeji-shi, Hyogo 671-1282 (JP)**

(74) Representative: **Wallinger Ricker Schlotter Tostmann**
**Patent- und Rechtsanwälte Partnerschaft mbB**
**Zweibrückenstraße 5-7**
**80331 München (DE)**

## (54) POLY(METH)ACRYLIC ACID (SALT) WATER-ABSORBING RESIN AND ABSORBENT ARTICLE

(57) An object lies in providing a water-absorbing resin capable of absorbing body fluids in a wide viscosity range from a body fluid having a low viscosity to a body fluid having a high viscosity, while maintaining water absorption capacity and water absorption speed. Used is a polyacrylic acid (salt)-based water-absorbing resin having: a free swell rate (A) with respect to an aqueous polyethylene oxide solution of 40°C of 0.15 $g \cdot g^{-1} \cdot s^{-1}$ or more; a free swell rate (B) with respect to a physiological saline of 40°C of 0.40 $g \cdot g^{-1} \cdot s^{-1}$ or more; and a free swell rate (A)/free swell rate (B) of 0.20 or more.

EP 4 338 832 A1

## Description

### Technical Field

The present invention relates to a water-absorbing resin and an absorbent body. More specifically, the present invention relates to a polyacrylic acid (salt)-based water-absorbing resin and an absorbent body.

### Background Art

In recent years, in hygienic materials such as a disposable diaper, a sanitary napkin, and an incontinence pad, a water-absorbing resin as a constituent material of the hygienic materials is widely utilized as a water-absorbing agent from the viewpoint of body fluid absorption. As such a water-absorbing resin, for example, a hydrolyzate of a starch-acrylonitrile graft copolymer, a neutralized product of a starch-acrylic acid graft polymer, a saponified product of a vinyl acetate-acrylic ester copolymer, a crosslinked product of a partially neutralized (meth)acrylic acid polymer, and the like are known. From the viewpoint of water absorption performance, a poly(meth)acrylic acid (salt)-based water-absorbing resin in which a (meth)acrylic acid and/or a salt thereof is/are used as a monomer(s) is industrially most often produced as a water-absorbing resin.

The performance required for a water-absorbing resin which is used in hygienic materials is exemplified by water absorption capacity and water absorption speed, from the viewpoint of body fluid absorption. These are generally parameters of absorption properties measured with use of a test liquid (for example, a 0.9 mass% aqueous sodium chloride solution) in imitation of urine (Non-patent Literature 1). Meanwhile, water-absorbing resins suitable for absorption of a body fluid(s) having a higher viscosity than urine, such as blood and/or loose stool, have been studied (Patent Literatures 1 to 3).

### Citation List

[Patent Literatures]

[Patent Literature 1]
Pamphlet of International Publication No. WO 2020/137241
[Patent Literature 2]
Pamphlet of International Publication No. WO 2002/085959
[Patent Literature 3]
Pamphlet of International Publication No. WO 2011/023572

[Non-patent Literature]

[Non-patent Literature 1]
Modern Superabsorbent Polymer Technology (published in 1998; Fredric L. Buchholz and Andrew T. Graham; publisher: WILEY-VCH)

### Summary of Invention

### Technical Problem

The viscosity of body fluids to be absorbed by hygienic materials (disposable diaper in particular) is not constant, for example, in a case where urine is excreted with loose stool. The conventional water-absorbing resin could not achieve both water absorption performance with respect to a body fluid having a low viscosity (for example, urine) and water absorption performance with respect to a body fluid having a high viscosity (for example, a mixture of urine and loose stool).

An object to be achieved by the present invention is to provide a water-absorbing resin capable of absorbing body fluids in a wide viscosity range, from a body fluid having a low viscosity (for example, urine) to a body fluid having a high viscosity (for example, a mixture of urine and loose stool), while maintaining a water absorption capacity of the water-absorbing resin and a water absorption speed thereof at a temperature near a body temperature of a human.

Solution to Problem

A water-absorbing resin in accordance with an embodiment of the present invention is a water-absorbing resin, wherein the water-absorbing resin is a polyacrylic acid (salt)-based water-absorbing resin, and the water-absorbing resin has: a free swell rate (A) with respect to an aqueous polyethylene oxide solution of 40°C of 0.15 $g \cdot g^{-1} \cdot s^{-1}$ or more; a free swell rate (B) with respect to a physiological saline of 40°C of 0.40 $g \cdot g^{-1} \cdot s^{-1}$ or more; and a free swell rate (A)/free swell rate (B) of 0.20 or more.

Advantageous Effects of Invention

According to an aspect of the present invention, it is possible to provide a water-absorbing resin that is excellent not only in water absorption speed with respect to a body fluid having a low viscosity (for example, urine) but also in water absorption speed with respect to a body fluid having a high viscosity (for example, a mixture of urine and loose stool), while maintaining a water absorption capacity of the water-absorbing resin and a water absorption speed thereof at a temperature near a body temperature of a human.

Description of Embodiments

The following description will discuss the present invention based on the best mode of the present invention. Any term used in the present specification should be understood as ordinarily used in this technical field unless particularly mentioned otherwise. Therefore, unless defined otherwise, all of the technical terms and scientific terms used in the present specification mean as generally understood by a person skilled in the technical field to which the present invention belongs. If there is any conflict in meaning, the present specification (including the definitions) take priority. The present invention is not limited to the embodiments described below and can be altered in various ways within the scope of the claims.

[1. Definitions of terms]

[1-1. Water-absorbing resin]

The term "water-absorbing resin" as used in an embodiment of the present invention refers to a waterswellable and water-insoluble polymer gelling agent that satisfies the following physical properties. Specifically, the term "water-absorbing resin" refers to a polymer gelling agent that satisfies the following physical properties: CRC (centrifuge retention capacity) defined in ERT 441.2-02 as "water-swelling property" is 5 g/g or more, and Ext (extractable) defined in ERT 470.2-02 as "water-insolubility" is 50 weight% or less.

The water-absorbing resin can be designed as appropriate according to its purpose of use, and is not limited to a particular design. The water-absorbing resin is preferably a hydrophilic crosslinked polymer that has been obtained by crosslinking and polymerizing unsaturated monomers each of which has a carboxyl group. The water-absorbing resin is not limited to a form in which the water-absorbing resin is wholly (that is, 100 weight%) a polymer, and can be in a form of a water-absorbing resin composition containing an additive and the like within a range in which the above-described physical properties (CRC and Ext) are satisfied.

The water-absorbing resin in an embodiment of the present invention may refer to not only an end product but also an intermediate produced during a process of producing the water-absorbing resin (e.g. a crosslinked hydrogel polymer after polymerization, a dried polymer after drying, a water-absorbing resin powder before surface crosslinking, and the like). All these water-absorbing resins, including the water-absorbing resin composition described above, will be collectively referred to as "water-absorbing resin". Examples of forms of the water-absorbing resin encompass a sheet form, a fiber form, a film form, a particulate form, and a gel form. The water-absorbing resin in an embodiment of the present invention is preferably a particulate water-absorbing resin.

[1-2. "EDANA" and "ERT"]

The term "EDANA" is an acronym for the European Disposables and Nonwovens Associations. The term "ERT" is an acronym for EDANA Recommended Test Methods, which are European standard (de facto international standard) measuring methods for a water-absorbing resin. In the present invention, physical properties of water-absorbing resin are measured in conformity with the ERT master copy (2002 revised version; known literature) unless otherwise specified.

[1-3. Others]

In the present specification, any range of "X to Y" means "X or more and Y or less".
In the present specification, "ppm" means "mass ppm" unless otherwise specified.
In the present specification, the unit of volume "liter" may be denoted as "l" or "L".
In the present specification, the terms "weight" and "mass" are used synonymously, the terms "weight%" and "mass%" are used synonymously, and the terms "parts by weight" and "parts by mass" are used synonymously.
In the present specification, the term "... acid (salt)" means "... acid and/or a salt thereof". The term "(meth)acrylic" means "acrylic and/or methacrylic". The term "polyacrylic acid (salt)-based water-absorbing resin" means a water-absorbing resin that contains, as a main component, a repeating unit derived from acrylic acid (salt), and specifically refers to a water-absorbing resin containing acrylic acid (salt) that accounts for preferably 50 mol% to 100 mol%, more preferably 70 mol% to 100 mol%, even more preferably 90 mol% to 100 mol%, and particularly preferably substantially 100 mol% of all monomers to be used for polymerization (excluding a crosslinking agent).

[2. Physical properties of water-absorbing resin]

"Shape of water-absorbing resin"

In an embodiment of the present invention, a water-absorbing resin is preferably in the form of particles, and specific examples of the shape of the particles encompass a non-uniformly pulverized shape, a spherical shape, a football shape, and an aggregate shape, and the like. Above all, a water-absorbing resin in the form of particles having a spherical shape has an increased bulk density, and a water-absorbing resin in the form of particles having an aggregate shape has an improved water absorption speed. Thus, the water-absorbing resin is more preferably an aggregate-like particle of spherical particles (for example, spherical particles including a polyacrylic acid (salt)-based water-absorbing resin). Note here that the spherical shape encompasses not only a true spherical shape but also a substantially spherical shape of an aspect ratio of 1.0 to 1.2.

"Additive to be contained"

In an embodiment of the present invention, an additive for allowing a water-absorbing resin to exhibit various functions can be contained. Specifically, examples of such an additive encompass a surfactant, a compound having a phosphorus atom, an oxidizer, an organic reducing agent, an inorganic reducing agent, water-insoluble inorganic fine particles, a chelating agent, a polyvalent metal salt, organic powder such as metallic soap, a deodorizing agent, an antibacterial agent, pulp, thermoplastic fibers, and the like. An amount of the additive used (added) is set as appropriate according to an application for which a resulting water-absorbing resin is used, and is 5 mass% or less, preferably 3 mass% or less, and more preferably 1 mass% or less, relative to the water-absorbing resin (for example, water-absorbing resin powder). A lower limit of the amount of the additive used (added) is 0.001 mass% or more and preferably 0.01 mass% or more relative to the water-absorbing resin (for example, water-absorbing resin powder). Note that, as the water-insoluble inorganic fine particles, a compound disclosed in "<5> Water-insoluble inorganic fine particles" of International Publication No. WO 2011/040530 is employed in an embodiment of the present invention. Among these water-insoluble inorganic fine particles, hydrophilic fine particles in particular, for example, silica (silicon dioxide) and/or hydrotalcite, are preferably contained because a resulting water-absorbing resin (for example, water-absorbing resin particles) has an improved compatibility with a liquid, and the water-absorbing resin, when used in an absorbent article, can absorb a water-based liquid in a short time. In addition, the polyvalent metal salt is preferably a water-soluble polyvalent metal salt and more preferably a water-soluble aluminum salt. Examples of the water-soluble aluminum salt encompass aluminum sulfate.
From the viewpoint of improving a compatibility of the water-absorbing resin (for example, water-absorbing resin particles) with a liquid, an amount of the water-insoluble inorganic fine particles added is 0.01 parts by mass to 5 parts by mass, more preferably 0.05 parts by mass to 3 parts by mass, and even more preferably 0.1 parts by mass to 1 part by mass, and particularly preferably 0.2 parts by mass to 0.5 parts by mass, relative to 100 parts by mass of the water-absorbing resin.

"CRC"

The term "CRC" is an acronym for centrifuge retention capacity and means a water absorption capacity without pressure of a water-absorbing resin.
The CRC of the water-absorbing resin in accordance with an embodiment of the present invention is preferably 25 g/g or more, more preferably 28 g/g or more, and even more preferably 31 g/g or more. The upper limit of the CRC

is not particularly limited, and a higher CRC is preferable. From the viewpoint of a balance with other physical properties, the upper limit of the CRC is preferably 50 g/g or less, more preferably 45 g/g or less, even more preferably 40 g/g or less, and particularly preferably 35 g/g or less.

A water-absorbing resin having a CRC of 25 g/g to 50 g/g achieves a sufficient absorption amount, prevents a decrease in the speed at which a body fluid or the like such as urine and/or blood is absorbed, and is suitable for use in, for example, a disposable diaper having a high water absorption speed. Note that the value of the CRC can be controlled by changing the type and/or amount of, for example, an internal crosslinking agent and/or a surface-crosslinking agent.

"Ext"

The term "Ext" is an abbreviation for "Extractables" (water-soluble component) and means an amount of water-soluble component extracted from a water-absorbing resin. The water-soluble component is measured in conformity with an EDANA method (ERT 470.2-02). In some cases, the measurement of the water-soluble component is carried out with the extraction time changed from 16 hours to 1 hour. In these cases, the water-soluble component is referred to as "Ext (1 hr)".

The Ext of the water-absorbing resin in accordance with an embodiment of the present invention is preferably 33 mass% or less, more preferably 30 mass% or less, and even more preferably 27 mass% or less. The lower limit of the Ext is 0 mass% or more. From the viewpoint of a balance with other physical properties, the lower limit of the Ext is preferably 2 mass% or more and more preferably 4 mass% or more.

A water-absorbing resin having an Ext of 33 mass% or less prevents or reduces a decrease in the speed at which a body fluid or the like such as urine and/or blood is absorbed and is suitable for use in, for example, a disposable diaper having a high water absorption speed. Note that the value of the Ext can be controlled by changing the type and/or amount of, for example, a polymerization initiator, an internal crosslinking agent, and/or a surface-crosslinking agent during production of a water-absorbing resin. The value of the Ext can also be controlled by using a chain transfer agent in the polymerization step during production of a water-absorbing resin.

The Ext (1 hr) of the water-absorbing resin in accordance with an embodiment of the present invention is preferably 15 mass% or less, more preferably 10 mass% or less, and even more preferably 9 mass% or less, 8 mass% or less, 7 mass% or less, 6 mass% or less, and 5 mass% or less in this order. The lower limit of the Ext (1 hr) is 0 mass% or more. From the viewpoint of a balance with other physical properties, the lower limit of the Ext (1 hr) is preferably 1 mass% or more and more preferably 2 mass% or more.

If the Ext (1 hr) exceeds 15 mass%, a polymer component extracted from a water-absorbing resin during water absorption may cause an increase in the viscosity of an absorbed liquid. Note that the value of the Ext (1 hr) is controlled not only by the method of controlling the Ext, but also by a method of mixing the composition of an aqueous surface-crosslinking agent solution and/or a water-absorbing resin.

"AAP"

The term "AAP" is an acronym for "absorption against pressure", and means a water absorption capacity under pressure of a water-absorbing resin. The AAP is measured in conformity with an EDANA method (ERT 442.2-02). Specifically, the AAP (absorption capacity under pressure) (unit: g/g) is measured after 0.9 g of water-absorbing resin has been allowed to swell for 1 hour under a load of 2.06 kPa (21g/$cm^2$, 0.3 psi) with use of a 0.9 mass% aqueous sodium chloride solution.

The AAP of the water-absorbing resin in accordance with an embodiment of the present invention is preferably 20 g/g or more and more preferably 25 g/g or more, from the viewpoint of water absorption property when used in hygienic materials. Further, the upper limit of the AAP of the water-absorbing resin is not particularly limited, and is preferably 45 g/g or less.

"Moisture content"

The "moisture content" is measured in conformity with an EDANA method (ERT 430.2-02), except that the amount of a sample is changed to 1.0 g, and a drying temperature is changed to 180°C.

The moisture content of the water-absorbing resin in accordance with an embodiment of the present invention is not particularly limited, and is preferably 1 mass% to 20 mass%, more preferably 1 mass% to 15 mass%, even more preferably 2 mass% to 13 mass%, and particularly preferably 2 mass% to 10 mass%. A water-absorbing resin having a moisture content of 1 mass% to 20 mass% prevents a decrease in the speed at which a body fluid or the like such as urine and/or blood is absorbed, and is suitable for use in, for example, a disposable diaper having a high water absorption speed.

"Mass average particle diameter (D50)"

The "mass average particle diameter (D50)" is measured in conformity with "(3) Mass-Average Particle Diameter (D50) and Logarithmic Standard Deviation (σζ) of Particle Diameter Distribution" described in Columns 27 and 28 of US Patent No. 7,638,570.

The mass average particle diameter (D50) of the water-absorbing resin in accordance with an embodiment of the present invention is preferably 200 pm to 700 pm, more preferably 250 pm to 600 pm, even more preferably 250 pm to 500 pm, and particularly preferably 300 pm to 450 pm. Further, the proportion of the particles having a particle diameter of less than 150 pm is preferably 20 mass% or less, more preferably 10 mass% or less, and even more preferably 5 mass% or less. A water-absorbing resin having a mass average particle diameter of 200 pm or more generates less dust and provides good handleability. Further, a water-absorbing resin having a mass average particle diameter of 700 pm or less prevents or reduces a decrease in the speed at which a body fluid or the like such as urine and/or blood is absorbed by the water-absorbing resin and is thus suitable for use in, for example, a disposable diaper having a high water absorption speed.

"Free swell rate (A) with respect to 3 weight% aqueous polyethylene oxide solution of 40°C"

The "free swell rate (A) with respect to 3 weight% aqueous polyethylene oxide solution of 40°C" means a water absorption speed (unit: $g \cdot g^{-1} \cdot s^{-1}$) at which a water-absorbing resin absorbs a 3 mass% aqueous polyethylene oxide solution of 40°C that is 20 times as heavy as its weight under no pressure and without stirring. The "free swell rate (A) with respect to 3 weight% aqueous polyethylene oxide solution of 40°C" may be referred to as "free swell rate (A)".

The free swell rate (A) of the water-absorbing resin in accordance with an embodiment of the present invention is preferably 0.15 $g \cdot g^{-1} \cdot s^{-1}$ or more, more preferably 0.18 $g \cdot g^{-1} \cdot s^{-1}$ or more, even more preferably 0.20 $g \cdot g^{-1} \cdot s^{-1}$ or more, and further preferably 0.25 $g \cdot g^{-1} \cdot s^{-1}$ or more. Further, the upper limit of the free swell rate (A) of the water-absorbing resin is not particularly limited and is preferably 0.50 $g \cdot g^{-1} \cdot s^{-1}$ or less.

"Free swell rate (B) with respect to physiological saline of 40°C"

The "free swell rate (B) with respect to physiological saline of 40°C" means a water absorption speed (unit: $g.g^{-1} \cdot s^{-1}$) at which a water-absorbing resin absorbs a physiological saline (0.9 mass% aqueous sodium chloride solution) of 40°C that is 20 times as heavy as its weight under no pressure and without stirring. The "free swell rate (B) with respect to physiological saline of 40°C" may be referred to as "free swell rate (B)".

The free swell rate (B) of the water-absorbing resin in accordance with an embodiment of the present invention is preferably 0.40 $g \cdot g^{-1} \cdot s^{-1}$ or more, more preferably 0.50 $g \cdot g^{-1} \cdot s^{-1}$ or more, and even more preferably 0.60 $g \cdot g^{-1} \cdot s^{-1}$ or more. Further, the upper limit of the free swell rate (B) of the water-absorbing resin is not particularly limited and is preferably 2.00 $g \cdot g^{-1} \cdot s^{-1}$ or less.

"Free swell rate ratio"

The "free swell rate ratio" is determined by free swell rate (A) [$g \cdot g^{-1} \cdot s^{-1}$]/free swell rate (B) [$g \cdot g^{-1} \cdot s^{-1}$].

The free swell rate ratio of the water-absorbing resin in accordance with an embodiment of the present invention is preferably 0.20 or more, more preferably 0.25 or more, and even more preferably 0.30 or more. Further, the upper limit of the free swell rate ratio of the water-absorbing resin is not particularly limited and is preferably 1.00 or less, more preferably 0.60 or less, and even more preferably 0.40 or less.

"Bulk density"

The "bulk density" is measured in conformity with an EDANA method (ERT 460.2-02).

The bulk density of the water-absorbing resin in accordance with an embodiment of the present invention is preferably 0.40 $g/cm^3$ to 0.80 $g/cm^3$, more preferably 0.50 $g/cm^3$ to 0.80 $g/cm^3$, and even more preferably 0.60 $g/cm^3$ to 0.75 $g/cm^3$. A water-absorbing resin having a bulk density of 0.40 $g/cm^3$ to 0.80 $g/cm^3$ prevents or reduces a decrease in the speed at which a body fluid or like such as urine and/or blood is absorbed by the water-absorbing resin and is thus suitable for use in, for example, a disposable diaper having a high water absorption speed.

"Number average particle diameter"

In a case where a water-absorbing resin has an aggregate shape, the number average particle diameter of the primary particles constituting the aggregate is measured with use of an electron microscope. The number average

particle diameter of the primary particles of the water-absorbing resin is preferably 5 pm to 1000 pm, more preferably 5 pm to 800 pm, even more preferably 8 pm to 500 pm, still more preferably 10 pm to 300 pm, further still more preferably 10 pm to 200 pm, and particularly preferably 30 pm to 100 pm.

"Surface tension"

The water-absorbing resin in accordance with an embodiment of the present invention has a surface tension of preferably 60 mN/m or more, more preferably 65 mN/m or more, even more preferably 67 mN/m or more, and particularly preferably 71 mN/m or more. There is no substantial decrease in surface tension. Ordinarily, 75 mN/m is sufficient as an upper limit of the surface tension.

[3. Method for producing water-absorbing resin]

A method for producing the water-absorbing resin in accordance with an embodiment of the present invention may use any of the following: aqueous solution polymerization, reversed phase suspension polymerization, vapor phase droplet polymerization, other polymerization methods, and the like. From the viewpoint of ease of control of the physical properties of the water-absorbing resin in accordance with an embodiment of the present invention, reversed phase suspension polymerization will be described below as an example. In particular, unlike general reversed phase suspension polymerization including an azeotropic dehydration step in a hydrophobic organic solvent after polymerization and/or a surface-crosslinking step in a dispersion system, a production method including a step of separating a gel obtained by reversed phase suspension polymerization, a gel sizing step, a drying step (preferably hot air drying), and a surface-crosslinking step (preferably powder surface treatment) will be described as an example.

In an embodiment of the present invention, the polymerization method only needs to be a polymerization method of obtaining a hydrogel polymer by reversed phase suspension polymerization in which a monomer is polymerized in a state in which droplets containing the monomer are dispersed or suspended in a liquid phase composed of a hydrophobic organic solvent. Such a polymerization method may be a batch-type method or a continuous-type method.

A batch-type production method is a production method in which an aqueous monomer solution is added or dropped into a hydrophobic organic solvent in a reaction apparatus and mixed to disperse or suspend the aqueous monomer solution, and then polymerization is carried out to obtain a hydrogel polymer.

On the other hand, a continuous-type production method is a method in which an aqueous monomer solution is continuously fed to a hydrophobic organic solvent in a reaction apparatus, dispersed or suspended, and then polymerized, and a hydrogel polymer formed by a polymerization reaction and the hydrophobic organic solvent are continuously discharged from the reaction apparatus.

A preferable embodiment of the present invention is continuous-type reversed phase suspension polymerization, and a more preferable embodiment thereof is liquid phase droplet continuous polymerization in which an aqueous monomer solution is continuously dispersed in a hydrophobic organic solvent to carry out polymerization. Such a continuous-type production process is preferable in that each operation in each step and between steps can be continuously carried out and that mass production becomes possible by long hours of operation. Further, the continuous-type reversed phase suspension polymerization is a preferable form from the viewpoint of the physical properties of a water-absorbing resin.

In the method for producing the water-absorbing resin in accordance with an embodiment of the present invention, a separation step of separating the hydrogel polymer obtained in the polymerization step from the hydrophobic organic solvent may be provided. In the continuous-type production process, the hydrophobic organic solvent separated from the hydrogel polymer in the separation step is preferably collected and reused as a hydrophobic organic solvent in the polymerization step. Such a circulation-type production process, which enables reduction of the amount of organic solvent used, is preferable in terms of production cost and waste liquid treatment.

Note that since continuous polymerization is a form in which an aqueous monomer solution is continuously suspended or dispersed as droplets in a hydrophobic organic solvent in a dispersion apparatus, and a dispersion and/or a suspension is/are continuously supplied to the reaction apparatus, the continuous polymerization is clearly distinguished from a form in which dispersion and polymerization are carried out in one apparatus (batch operation, batch type). In addition, in a case where the operation is continuously carried out, the operation time is preferably 1 hour or longer, more preferably 3 hours or longer, even more preferably 8 hours or longer, and further preferably 24 hours or longer. Further, the operation time is usually one year or shorter.

A method for producing a water-absorbing resin in accordance with an embodiment of the present invention includes: any aqueous monomer solution preparation step; any dispersion step; a polymerization step; any separation step; any gel sizing step; and a drying step. Further, the method can optionally include, after the drying step, a cooling step, a pulverizing step, a classification step, a surface-crosslinking step, a re-moistening step, a sizing step, a fine powder removal step, a granulation step, a fine powder reuse step, and the like. Further, the method may further include

a conveying step, a storing step, a packing step, a reserving step, and the like.

The individual steps will be described below.

[3-1: Aqueous monomer solution preparation step]

The aqueous monomer solution is an aqueous solution that contains a monomer which serves as a raw material for a water-absorbing resin, and is a solution to be dispersed or suspended in a hydrophobic organic solvent in order to carry out reversed phase suspension polymerization.

As the solvent of the aqueous monomer solution, water or a mixture of water and a water-soluble organic solvent (for example, alcohol or the like) is suitably used. The solvent of the aqueous monomer solution is even more preferably water. In a case where the solvent is a mixture of water and a water-soluble organic solvent, the water-soluble organic solvent (for example, alcohol or the like) accounts for preferably 30 mass% or less and more preferably 5 mass% or less of the mixture.

As the monomer, a water-soluble ethylenically unsaturated monomer is preferably used. Examples of the water-soluble ethylenically unsaturated monomer encompass: acid group-containing unsaturated monomers such as (meth)acrylic acid, maleic acid (anhydride), itaconic acid, cinnamic acid, vinylsulfonic acid, allyltoluene sulfonic acid, vinyltoluene sulfonic acid, styrene sulfonic acid, 2-(meth)acrylamide-2-methylpropane sulfonic acid, 2-(meth)acryloylethane sulfonic acid, 2-(meth)acryloylpropane sulfonic acid, 2-hydroxyethyl (meth)acrylate, 2-hydroxypropyl (meth)acrylate, 2-hydroxyethyl (meth)acryloyl phosphate, methoxypolyethylene glycol (meth)acrylate, and polyethylene glycol mono(meth)acrylate; amide group-containing unsaturated monomers such as (meth)acrylamide, N-ethyl (meth)acrylamide, N,N-dimethyl (meth)acrylamide, N-n-propyl (meth)acrylamide, N-isopropyl (meth)acrylamide, vinylpyridine, N-vinylpyrrolidone, N-acrylloyl piperidin, N-acrylloyl pyrrolidine, and N-vinylacetamide; amino group-containing unsaturated monomers such as N,N-dimethylaminoethyl (meth)acrylate, N,N-dimethylaminopropyl (meth)acrylate, N,N-dimethylaminopropyl (meth)acrylamide, and N,N-diethylaminoethyl (meth)acrylate; mercapto group-containing unsaturated monomers; phenolic hydroxyl group-containing unsaturated monomers; lactam group-containing unsaturated monomers such as N-vinylpyrrolidone; and the like.

Note that, in consideration of stability of the water-soluble ethylenically unsaturated monomer, a polymerization inhibitor may be added, as necessary, to the aqueous monomer solution.

In a case where an acid group-containing unsaturated monomer having an acid group such as a carboxyl group among the water-soluble ethylenically unsaturated monomers is used to produce a water-absorbing resin, a neutralized salt obtained by neutralizing the acid group can be used. In this case, a salt of the acid group-containing unsaturated monomer is preferably a salt with a monovalent cation, more preferably at least one salt selected from the group consisting of an alkali metal salt, an ammonium salt, and an amine salt, further preferably an alkali metal salt, even further preferably at least one salt selected from the group consisting of a sodium salt, a lithium salt, and a potassium salt, and particularly preferably a sodium salt.

Among these water-soluble ethylenically unsaturated monomers, from the viewpoint of the water absorption performance of a resulting water-absorbing resin, the water-soluble ethylenically unsaturated monomer is preferably an acid group-containing unsaturated monomer and/or a salt thereof, more preferably (meth)acrylic acid (salt), maleic acid (anhydride) (salt), itaconic acid (salt), and cinnamic acid (salt), further preferably (meth)acrylic acid (salt), and particularly preferably acrylic acid (salt).

In a case where an acid group-containing unsaturated monomer is used as the monomer, the acid group-containing unsaturated monomer and a neutralized salt of the acid group-containing unsaturated monomer are preferably used in combination from the viewpoint of water absorption performance of a resulting water-absorbing resin. From the viewpoint of water absorption performance, the number of moles of the neutralized salt relative to the total number of moles of the acid group-containing unsaturated monomer and the neutralized salt thereof (hereinafter, referred to as "neutralization ratio") is preferably 40 mol% or more, more preferably 40 mol% to 95 mol%, further preferably 50 mol% to 90 mol%, even further preferably 55 mol% to 85 mol%, and particularly preferably 60 mol% to 80 mol%.

In the production method in accordance with an embodiment of the present invention, in the preparation of the aqueous monomer solution, any of the monomers listed above as examples may be used solely, or any two or more of the monomers may be mixed as appropriate and used. In addition, another monomer may be further mixed and used, provided that the object of the present invention is achieved.

In a case where two or more of the monomers are used in combination in the preparation of the aqueous monomer solution, it is preferable that the monomers used for the polymerization include acrylic acid (salt) (for example, (meth)acrylic acid (salt)) as a main component. In this case, from the viewpoint of the water absorption performance of a resulting water-absorbing resin, the proportion of the acrylic acid (salt) to the entire monomer used for the polymerization is normally 50 mol% or more, preferably 70 mol% or more, more preferably 80 mol% or more, and further preferably 90 mol% or more (the upper limit is 100 mol%).

In the preparation of the aqueous monomer solution, an internal crosslinking agent can be used as necessary.

The internal crosslinking agent is exemplified by a conventionally known internal crosslinking agent having two or more polymerizable unsaturated groups and/or two or more reactive groups within one molecule thereof. Examples of the internal crosslinking agent encompass N,N-methylene bis(meth)acrylamide, (poly)ethylene glycol di(meth)acrylate, (poly)propylene glycol di(meth)acrylate, trimethylol propane tri(meth)acrylate, trimethylol propane di(meth)acrylate, glycerin tri(meth)acrylate, glycerin acrylate methacrylate, ethylene oxide-modified trimethylol propane tri(meth)acrylate, pentaerythritol tetra(meth)acrylate, dipentaerythritol hexa(meth)acrylate, triallyl cyanurate, triallyl isocyanurate, triallyl phosphate, triallylamine, poly(meth)allyloxy alkane, (poly)ethylene glycol diglycidyl ether, glycerol diglycidyl ether, ethylene glycol, polyethylene glycol, propylene glycol, glycerin, 1,4-butanediol, pentaerythritol, ethylenediamine, ethylene carbonate, propylene carbonate, polyethyleneimine, glycidyl (meth)acrylate, and the like. Only one of these internal crosslinking agents may be used solely, or two or more thereof may be used in combination.

The amount of the internal crosslinking agent used is determined as appropriate in accordance with the physical properties of a desired water-absorbing resin and is normally 0.0001 mol% to 5 mol%, more preferably 0.001 mol% to 3 mol%, and even more preferably 0.005 mol% to 1.5 mol%, relative to the monomer.

In addition, substances (hereinafter, referred to as "other substances") examples of which will be listed below can also be added to the aqueous monomer solution.

Specific examples of the other substances encompass: chain transfer agents such as thiols, thiolic acids, secondary alcohols, amines, and hypophosphites; foaming agents such as carbonates, bicarbonates, azo compounds, and bubbles; chelating agents such as metal salts of ethylenediamine tetraacetic acid, and metal salts of diethylenetriamine pentaacetic acid; polyacrylic acid (salt) and crosslinked products thereof; starch; cellulose; starch-cellulose derivatives; polyvinyl alcohol; and the like. The other substances may be used solely, or two or more of the other substances may be used in combination.

The amount of the other substances used is not particularly limited, and the total concentration of the other substances is preferably 10 mass% or less, more preferably 1 mass% or less, and even more preferably 0.1 mass% or less, relative to the monomer. However, the total concentration of polyacrylic acid (salt) and crosslinked products thereof, starch, cellulose, starch-cellulose derivatives, or polyvinyl alcohol is preferably 30 mass% or less, more preferably 20 mass% or less, and even more preferably 10 mass% or less, relative to the monomer.

Further, dissolved oxygen in the aqueous monomer solution may be reduced by temperature increase or substitution with an inert gas.

"Polymerization initiator"

In the preparation of the aqueous monomer solution, a polymerization initiator may be used. Note that, in a case where a polymerization initiator is used in the preparation of an aqueous monomer solution, gelation of the aqueous monomer solution and/or increase in viscosity thereof may occur. Thus, for the addition of the polymerization initiator to the aqueous monomer solution, for example, the following (1), (2), and (3) are preferably carried out: (1) the addition of the polymerization initiator is carried out immediately before the aqueous monomer solution is dispersed and/or suspended in a hydrophobic organic solvent; (2) the aqueous monomer solution is cooled and is mixed with the polymerization initiator at a temperature (20°C or lower, preferably around 0°C) lower than normal temperature; and (3) the dispersion step is carried out while the aqueous monomer solution and the polymerization initiator are mixed in a static mixer. As the polymerization initiator, a pyrolytic polymerization initiator is preferably used. The pyrolytic polymerization initiator refers to a compound that is decomposed by heat to generate radicals. From the viewpoint of storage stability of the pyrolytic polymerization initiator and/or production efficiency of a water-absorbing resin, preferably used as the polymerization initiator is a water-soluble compound having a 10-hour half-life temperature of preferably 0°C to 120°C, more preferably 30°C to 100°C, and even more preferably 50°C to 80°C.

From the viewpoint of the handleability of the pyrolytic polymerization initiator and/or the physical properties of a water-absorbing resin, used as the polymerization initiator are preferably azo compounds and persulfates, more preferably sodium persulfate, potassium persulfate, and ammonium persulfate, and further preferably sodium persulfate.

The amount of the pyrolytic polymerization initiator used is set as appropriate in accordance with, for example, the types of the monomer and of the polymerization initiator and is not particularly limited. From the viewpoint of production efficiency, the amount of the pyrolytic polymerization initiator used is preferably 0.001 g/mol or more, more preferably 0.005 g/mol or more, and even more preferably 0.010 g/mol or more, relative to the monomer. In addition, from the viewpoint of improvement in water absorption performance of a water-absorbing resin, the amount of the pyrolytic polymerization initiator used is preferably 2 g/mol or less and more preferably 1 g/mol or less.

In addition, if necessary, the pyrolytic polymerization initiator can be used in combination with another polymerization initiator such as a photolytic polymerization initiator. Specific examples of the photolytic polymerization initiator encompass benzoin derivatives, benzyl derivatives, acetophenone derivatives, benzophenone derivatives, and the like.

In addition, the pyrolytic polymerization initiator and a reducing agent can be used in combination as a redox polymerization initiator. In the redox polymerization initiator, the pyrolytic polymerization initiator functions as an oxidizing

agent. The reducing agent to be used is not particularly limited, and examples of the reducing agent encompass: (bi)sulfites such as sodium sulfite and sodium hydrogen sulfite; reducing metal salts such as a ferrous salt; L-ascorbic acid (salt); amines; and the like.

"Concentration of monomer in aqueous monomer solution"

In an embodiment of the present invention, the concentration of a monomer in an aqueous monomer solution is selected according to, for example, the types of a selected monomer and of a selected hydrophobic organic solvent. In terms of production efficiency, the lower limit of the concentration of the monomer in the aqueous monomer solution (100 mass%) is preferably 10 mass% or more, more preferably 20 mass% or more, and even more preferably 30 mass% or more in the aqueous monomer solution, and the upper limit thereof is preferably 100 mass% or less, more preferably 90 mass% or less, even more preferably 80 mass% or less, and further more preferably 70 mass% or less.

It is also possible to blend an additive such as an internal crosslinking agent, a surfactant, a density adjusting agent, a thickener, and a chelating agent into the aqueous monomer solution, provided that the object of an embodiment of the present invention is not impaired. Note that the type of the additive and the amount of the additive added can be selected as appropriate depending on a combination of the monomer used and the hydrophobic organic solvent used.

[3-2: Dispersion step]

The dispersion step is a step of dispersing or suspending droplets containing a monomer in a hydrophobic organic solvent. Note that, hereinafter, in a case where the term "dispersion" is simply described, the dispersion conceptually encompasses suspension. More specifically, the aqueous monomer solution is added to a hydrophobic organic solvent, mixed, and stirred so that the aqueous monomer solution is dispersed in the hydrophobic organic solvent. For example, a stirring apparatus provided with a stirring blade (such as a propeller blade, a paddle blade, an anchor blade, a turbine blade, a Phaudler blade, a ribbon blade, and a flat plate blade) may be used. In a case where a stirring apparatus having such a stirring blade is used, a dispersed droplet diameter can be adjusted depending on the type of the stirring blade, a blade diameter, the number of rotations, and the like. The stirring apparatus having such a stirring blade can be particularly suitably used when batch-type reversed phase suspension polymerization is carried out. Further, a dispersion can be obtained by a method described in, for example, International Publication No. WO 2009/025235 and International Publication No. WO 2013/018571. In a case where the continuous-type reversed phase suspension polymerization is carried out, the dispersion step is preferably such that the aqueous monomer solution and the hydrophobic organic solvent are continuously and separately supplied to the dispersion apparatus, and droplets containing the monomer and dispersed in the hydrophobic organic solvent are prepared.

In a case where the continuous-type reversed phase suspension polymerization is carried out, examples of the dispersion apparatus used in the dispersion step encompass: a spray nozzle; a high-speed rotary shear type stirrer (such as a rotary mixer type, a turbo mixer type, a disk type, and a double cylinder type); a cylindrical nozzle such as a needle; an orifice plate in which a large number of holes are directly provided in the plate; a centrifugal atomizer such as a rotary wheel; and the like. However, there is no particular limitation.

"Hydrophobic organic solvent"

A preferable hydrophobic organic solvent is exemplified by at least one organic solvent selected from the group consisting of aliphatic hydrocarbons, alicyclic hydrocarbons, aromatic hydrocarbons, and halogenated hydrocarbons. Specific examples of the hydrophobic organic solvent encompass: aliphatic hydrocarbons such as n-pentane, n-hexane, n-heptane, and n-octane; alicyclic hydrocarbons such as cyclohexane, methylcyclohexane, cyclooctane, and decalin; aromatic hydrocarbons such as benzene, toluene, and xylene; and halogenated hydrocarbons such as chlorobenzene, bromobenzene, carbon tetrachloride, and 1,2-dichloroethane. Among these hydrophobic organic solvents, from the viewpoint of ease of availability and quality stability, the hydrophobic organic solvent is preferably one or more selected from the group consisting of n-hexane, n-heptane, and cyclohexane. The hydrophobic organic solvent can also be used as a mixture solvent in which two or more of these hydrophobic organic solvents are mixed.

In an embodiment of the present invention, a dispersion aid such as a surfactant and/or a polymer additive may be added, as necessary, to the hydrophobic organic solvent, provided that the object of an embodiment of the present invention is not impaired. The type of dispersion aid is selected as appropriate depending on a combination of the hydrophobic organic solvent and the monomer to be used, and examples of the dispersion aid that can be used encompass the following surfactants and polymer additives.

Specific examples of the surfactant encompass sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers,

polyoxyethylene castor oil, polyoxyethylene hardened castor oil, alkylallylformaldehyde-condensed polyoxyethylene ethers, polyoxyethylene polyoxypropylene block copolymers, polyoxyethylene polyoxypropyl alkyl ethers, polyethylene glycol fatty acid esters, alkyl glucosides, N-alkyl gluconamides, polyoxyethylene fatty acid amides, polyoxyethylene alkylamines, phosphates of polyoxyethylene alkyl ethers, phosphates of polyoxyethylene alkyl aryl ethers, and the like. One of these surfactants may be used solely, or two or more thereof may be used in combination. Further, a polymerizable surfactant having polymerizability can also be used. The polymerizable surfactant is, specifically, exemplified by a compound having the following structure:

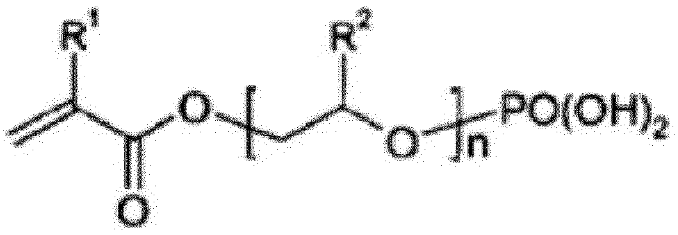

Note that, in the formula, $R^1$ and $R^2$ are hydrogen, methyl, or ethyl independently of each other, and n means an integer of 3 to 20. Among the above-described surfactants, fatty acid esters such as sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, and polyethylene glycol fatty acid esters are preferable. Among them, sucrose fatty acid esters are preferable. In addition, a hydrophilelipophile balance (HLB) of the surfactant used in an embodiment of the present invention is not particularly limited, and is in the range of preferably 1 to 20, more preferably 1 to 10, and even more preferably 3 to 6.

Specific examples of the polymer additive encompass maleic anhydride-modified polyethylene, maleic anhydride-modified polypropylene, maleic anhydride-modified ethylene-propylene copolymer, maleic anhydride-modified ethylene-propylene-diene terpolymer (EPDM), maleic anhydride-modified polybutadiene, maleic anhydride-ethylene copolymer, maleic anhydride-propylene copolymer, maleic anhydride-ethylene-propylene copolymer, maleic anhydridebutadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, hydroxyethyl cellulose, and the like. Among them, from the viewpoint of dispersion stability of the aqueous monomer solution, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are preferable. One of these polymer additives may be used solely, or two or more thereof may be used in combination. In addition, these polymer additives and the surfactant may be used in combination. Above all, it is preferable to use the polymer additive, and it is more preferable to use a maleic anhydride-modified ethylene-propylene copolymer. Further, in another preferred embodiment, the polymer additive is used solely without using the surfactant.

The amount of the dispersion aid used is set as appropriate in accordance with, for example, the polymerization form and the types of the aqueous monomer solution and of the hydrophobic organic solvent. Specifically, the concentration of the dispersion aid in the hydrophobic organic solvent is preferably 0.0001 mass% to 2 mass% and more preferably 0.0005 mass% to 1 mass%.

[3-3. Polymerization step]

The polymerization step is a step of polymerizing droplets containing the monomer obtained in the dispersion step so that a hydrogel polymer (hereinafter also referred to simply as "hydrogel") is obtained.

"Reaction apparatus"

As the reaction apparatus to be used in the polymerization step, the dispersion apparatus used in the dispersion step may be used as it is, or a different apparatus may be used. In the case of the batch-type reversed phase suspension polymerization, the apparatus used in the dispersion step can be used as it is as the reaction apparatus. This is suitable in terms of workability. In a case where the reaction apparatus is an apparatus different from the dispersion apparatus, the dispersion liquid of the monomer obtained in the dispersion step is supplied to the reaction apparatus.

Further, the shape of the reaction apparatus in which the polymerization reaction is carried out is not particularly limited, and a known reaction apparatus can be used. As described above, the stirring apparatus that can be suitably

used in the dispersion step can also be suitably used in the polymerization reaction. In the case of the continuous-type production method, the shape of the reaction apparatus is preferably a shape that allows the monomer (aqueous solution) to undergo polymerization reaction while moving, as a droplet-like dispersed phase, in a hydrophobic organic solvent which is a continuous phase formed in this reaction apparatus. Examples of such a reaction apparatus encompass a reaction apparatus in which a tubular reaction tube is disposed in a vertical fashion, a horizontal fashion, or a spiral fashion. In this aspect, since the monomer (aqueous solution) is supplied into the hydrophobic organic solvent that moves in the reaction unit, the droplets composed of the aqueous monomer solution move together with the hydrophobic organic solvent without staying. This prevents contact between monomer reactants having different polymerization rates.

Further, the reaction apparatus may be provided with a temperature adjustment means so that the continuous phase inside the reaction apparatus can be heated and/or cooled from the outside, if necessary.

"Polymerization temperature"

A polymerization temperature, which is a reaction temperature in the polymerization step, is set as appropriate depending on the type and/or amount of the polymerization initiator to be used, and is preferably 20°C to 100°C and more preferably 40°C to 90°C. The polymerization temperature of higher than 100°C is not preferable because such a polymerization temperature causes a rapid polymerization reaction. Note that the polymerization temperature means the temperature (hereinafter referred to as "Td") of the hydrophobic organic solvent that serves as a dispersion medium.

In the polymerization step, the monomer (aqueous solution) is dispersed in the hydrophobic organic solvent in the form of droplets. Thus, the temperature of the aqueous monomer solution rapidly increases due to heat transfer from the hydrophobic organic solvent. In a case where the polymerization initiator contained in the droplets is a pyrolytic polymerization initiator, the pyrolytic polymerization initiator is decomposed with the increase in temperature, so that radicals are generated. Then, the polymerization reaction is initiated by the generated radicals, and a hydrogel is formed as the polymerization reaction progresses.

In the case of the continuous-type production method, the formed hydrogel is moved inside the reaction apparatus by the moving continuous phase, and is discharged from the reaction apparatus together with the hydrophobic organic solvent that forms the continuous phase.

In a case where the aqueous monomer solution contains a pyrolytic polymerization initiator, the Td is preferably 70°C or higher, more preferably 75°C or higher, and even more preferably 80°C or higher, from the viewpoint of the polymerization rate. The upper limit of Td is not particularly limited and is selected as appropriate, from the viewpoint of safety, in a range which does not exceed the boiling point of the hydrophobic organic solvent that forms the continuous phase.

"Multi-stage reversed phase suspension polymerization"

In the production method in accordance with an embodiment of the present invention, multi-stage polymerization may be carried out from the viewpoint of obtaining an appropriate aggregated particle diameter. Specifically, the multi-stage polymerization can be carried out, for example, by, after the end of a first-stage polymerization step, further adding an aqueous monomer solution and carrying out a polymerization reaction.

"Inorganic fine particles"

In the production method in accordance with an embodiment of the present invention, inorganic fine particles may be added to the hydrogel polymer during the polymerization and/or after the end of the polymerization, from the viewpoint of obtaining an appropriate aggregated particle diameter.

Examples of the inorganic fine particles that can be used in an embodiment of the present invention encompass silicon dioxide, amorphous silica, aluminum oxide, titanium dioxide, calcium phosphate, calcium carbonate, magnesium phosphate, calcium sulfate, diatomaceous earth, bentonite, zeolite, other metal oxides, and the like. As the inorganic fine particles, silicon dioxide, aluminum oxide, and titanium dioxide in particular, are preferable.

Good results can be obtained when the amount of the inorganic fine particles added is such that the inorganic fine particles are used in a proportion of generally 0.001 parts by weight to 1 part by weight, preferably 0.001 parts by weight to 0.5 parts by weight, relative to the hydrogel polymer. The amount of the inorganic fine particles added falling within this range is preferable since the effect produced by the addition of the inorganic fine particles is efficiently exhibited, and the influence on the water absorption performance is small.

[3-4. Separation step]

The separation step is a step of separating the hydrogel polymer obtained in the polymerization step from the

hydrophobic organic solvent. The type and structure of the apparatus to be used in the separation step are not particularly limited. For example, a known apparatus used for filtration, sedimentation, centrifugation, squeezing, and the like can be used. Further, separation of the hydrogel polymer from the hydrophobic organic solvent may be carried out by distillation performed through the heating of a mixture of the hydrogel polymer and the hydrophobic organic solvent under normal pressure or reduced pressure with use of the stirring apparatus having a stirring blade and used in the polymerization step. In the batch-type reversed phase suspension polymerization, distillation under normal pressure or reduced pressure is suitably performed.

"Solvent content percentage of hydrophobic organic solvent in hydrogel"

A solvent content percentage of the hydrophobic organic solvent in the hydrogel separated from the hydrophobic organic solvent through the separation step is not particularly limited. However, from the viewpoint of the load during drying and/or the cost of the organic solvent, the solvent content percentage of the hydrophobic organic solvent (hereinafter also referred to simply as solvent content percentage) is preferably 0.01 mass% to 10 mass%, more preferably 0.01 mass% to 9 mass%, and even more preferably 0.01 mass% to 5 mass%, relative to 100 mass% of the hydrogel including the solvent.

"Percentage of solid content in hydrogel polymer"

A percentage of a solid content in the hydrogel separated from the hydrophobic organic solvent is not particularly limited. However, the percentage of the solid content in the hydrogel polymer is preferably 20 mass% or more, more preferably 30 mass% or more, and even more preferably 40 mass% or more, from the viewpoint of the drying cost in the drying step which is a subsequent step. Further, the upper limit of the percentage of the solid content in the hydrogel polymer is preferably 90 mass% or less, more preferably 80 mass% or less, even more preferably 70 mass% or less, and particularly preferably 60 mass% or less, from the viewpoint of water absorption performance and/or mechanical load.

"Gel polymerization rate"

A gel polymerization rate of the obtained hydrogel is preferably 70 mass% or more, more preferably 80 mass% or more, even more preferably 90 mass% or more, and particularly preferably 95 mass% or more, from the viewpoint of preventing aggregation of the obtained hydrogel during drying and reducing residual monomers in a resulting water-absorbing resin. Ideally, the upper limit of the gel polymerization rate is 100 mass%. The polymerization rate of 70 mass% or more makes it possible to prevent hydrogels from strongly aggregating with each other and agglomerating during drying.

[3-5. Gel sizing step]

In the gel sizing step, the hydrogel polymer separated from the hydrophobic organic solvent in the separation step is sized with use of a gel sizing apparatus having an extrusion unit and a porous plate. As a result, a sized hydrogel polymer (hereinafter, the hydrogel after gel sizing is referred to as sized gel) is obtained. The gel sizing step is an optional step. Having the gel sizing step makes it easy to control the water absorption speed of a high-viscosity liquid.

The hydrogel polymer used in the gel sizing step is in the shape of a single particle of a spherical gel or in the shape of an aggregate of spherical gels. The lower limit of the average particle diameter of the hydrogel polymer is not particularly limited and is preferably 0.01 mm or more, more preferably 0.03 mm or more, even more preferably 0.05 mm or more, still more preferably 0.1 mm or more. The upper limit of the average particle diameter of the hydrogel polymer is also not particularly limited and is preferably 20 mm or less and more preferably 10 mm or less. Further, in the case where the hydrogel polymer is in the form of a single particle, the particle diameter of the spherical gel is referred to as a primary particle diameter. In the case where the hydrogel polymer is in the form of an aggregate, the particle diameter of each spherical gel constituting the aggregate is referred to as a primary particle diameter. In an embodiment of the present invention, an average primary particle diameter is not particularly limited, and is preferably 5 pm to 2000 pm, more preferably 5 pm to 1000 pm, even more preferably 5 pm to 800 pm, still more preferably 8 pm to 500 pm, further more preferably 10 pm to 300 pm, and particularly preferably 10 pm to 200 pm, from the viewpoint of preventing the generation of fine powder when control is carried out to have the particle size of a final product.

Note that an apparatus having a cutter may be installed in front of the gel sizing apparatus having the extrusion unit and the porous plate to crush large aggregates.

"Gel sizing apparatus"

In the present specification, "gel sizing" means an operation of extruding a wet powder mass (for example, a

hydrogel) into a columnar shape through a small hole of a porous plate to prepare grains having a substantially uniform shape and size from a wet powder-like raw material. That is, with use of the porous plate, hydrogels in the form of a coarse aggregate resulting from excessive aggregation in a solvent separation step which is a previous step are crushed, and hydrogels in the form of a single particle having a small particle diameter are appropriately aggregated. Therefore, the gel sizing step makes it possible to obtain a hydrogel (sized gel) having a granulation shape and having a relatively uniform particle diameter. The sized gel may contain a hydrogel in the form of a single particle.

As the "gel sizing apparatus having an extrusion action unit and a porous plate" used in the gel sizing step is not particularly limited, provided that the gel sizing apparatus is an apparatus (for example, an extruder) having an extrusion action unit and a porous plate (die or screen), the extrusion action unit generally having an extrusion member for extruding and supplying the contents toward the porous plate, and being capable of preparing grains of a certain size by extruding a material from the porous plate. Further, these apparatuses may be placed in series and used.

Further, the shape of the hole of this porous plate (die or screen) is not particularly limited, and it is possible to freely select a shape suitable for use, including a perfect-circular shape, an elliptical shape, a polygonal shape such as a hexagonal shape, a triangular shape, and the like. From the viewpoint of sizing strength, a perfect-circular shape and an elliptical shape are preferable. The hole diameter is also not particularly limited and is preferably 1.5 mm or less, more preferably 1.0 mm or less, and even more preferably 0.8 mm or less. Such an upper limit prevents or reduces an increase in the size of a resulting sized gel more than necessary, and makes it possible to reduce the amount of fine powder generated when control is carried out to have the particle size of a final product. The hole diameter is preferably 0.3 mm to 1.5 mm and more preferably 0.3 mm to 0.8 mm. With the porous plate having a pore diameter of 0.3 mm or more, it is possible to efficiently carry out extrusion when an extrusion operation is carried out. Note that the hole diameter is defined as follows. First, in a case where the hole does not have a perfect-circular shape, a geometric mean value of a minor axis of the hole and a major axis thereof is adopted as the hole diameter. Further, in a case where the holes of the porous plate have different hole diameters, the hole diameters of all the holes are calculated, and an arithmetic mean value of the hole diameters is adopted as the hole diameter of the holes of the porous plate. Furthermore, in a case where the porous plate has varying hole diameters in a distance between an extrusion action unit side of the porous plate and a side opposite to the extrusion action unit side (the hole diameter varies in the thickness direction of the porous plate), a smallest hole diameter value among the hole diameters is adopted.

In the gel sizing step, an additive may be further added. Examples of the additive that can be added in the gel sizing step encompass a polymerization initiator, an oxidizing agent, a reducing agent, a chelating agent, a thickener, a surfactant, a crosslinking agent, an acid, a base, a foaming agent, organic or inorganic fine particles, a polyvalent metal salt, and the like. Among them, preferable as an additive that can control the degree of aggregation are, for example, a thickener such as starch, cellulose, a starch-cellulose derivative, and polyvinyl alcohol, a surfactant, fine powder of a water-absorbing resin, a crosslinking agent, a polyvalent metal salt, and the like. The polyvalent metal salt is preferably a water-soluble polyvalent metal salt and more preferably a water-soluble aluminum salt. Examples of the water-soluble aluminum salt encompass aluminum sulfate.

[3-6. Drying step]

The drying step is a step of drying a hydrogel. As a result, moisture contained in the hydrogel and the hydrophobic organic solvent that has not be completely separated in the optional separation step are removed, so that a particulate dried polymer having a solid content in a desired percentage is obtained. The percentage of a solid content in the dried polymer is preferably 80 weight% or more, more preferably 85 weight% to 99 weight%, even more preferably 90 weight% to 98 weight%, and particularly preferably 92 weight% to 97 weight%.

In an embodiment of the present invention, a drying method is not particularly limited. Examples of the drying method encompass thermal drying, hot air drying, drying under reduced pressure, fluidized bed drying, infrared drying, microwave drying, drum dryer drying, drying by azeotropic dehydration with a hydrophobic organic solvent, high humidity drying utilizing high temperature water vapor, and the like.

From the viewpoint of a color tone of a water-absorbing resin and/or drying efficiency, the hot air drying is performed at a drying temperature (temperature of hot air) of preferably 100°C to 250°C and more preferably 100°C to 180°C. Note that drying conditions, such as an air velocity of hot air and/or a drying time, other than the drying temperature can be set as appropriate in accordance with a moisture content of a particulate hydrogel to be dried, a total weight thereof, and a desired resin solid content. In the case of band drying, various conditions disclosed in, for example, International Publication No. WO 2006/100300, International Publication No. WO 2011/025012, International Publication No. WO 2011/025013, and International Publication No. WO 2011/111657 can be employed as necessary.

The dried polymer composed of particles obtained in this drying step can be directly used as a water-absorbing resin for each application. Further, in a case where a water-absorbing resin is produced by this production method, the dried polymer obtained in the drying step can be subjected to the surface-crosslinking step described later. In this case, the dried polymer to be subjected to the surface-crosslinking step described later is also referred to as "water-absorbing

resin powder" for convenience. In a case where a dried product in which the sized gels are aggregated is obtained in the drying step, it is preferable that the drying step encompasses a step of disintegrating an aggregate of the sized gels.

"Additive"

An additive may be added to a hydrogel, provided that the effect of an embodiment of the present invention is not impaired. The additive may be added during heating by a heating means or may be carried out before the drying step (before heating by the heating means). Furthermore, the addition may be carried out in any step previous to the drying step. With the additive, excessive adhesion between hydrogels during drying can be reduced, and a water-absorbing resin having an excellent water absorption speed can be obtained.

[3-7. Hydrophilization treatment step]

In an embodiment of the present invention, the hydrophilization treatment step may be carried out after the surface-crosslinking step described later or before the surface-crosslinking step. The hydrophilization treatment step is a step of subjecting a water-absorbing resin to hydrophilization treatment with an organic solvent. This makes it possible to enhance the effect of surface-crosslinking treatment (the effect of increasing absorption capacity of a water-absorbing resin under pressure and reducing a re-wet). In addition, since the surface of the water-absorbing resin becomes highly hydrophilic, the water absorption speed also increases.

A specific method for the hydrophilization treatment varies depending on the cause of the formation of a hydrophobic portion on the surface of a water-absorbing resin. For example, in a case where a water-absorbing resin has been produced by reversed phase suspension polymerization, a surfactant used as a dispersant during polymerization is the cause of hydrophobicity. Thus, it is preferable to clean the surfactant, and it is more preferable to clean the surfactant with use of an organic solvent.

The organic solvent is not particularly limited, provided that the organic solvent is capable of cleaning the surfactant. In order to enhance a cleaning effect, it is preferable to use an organic solvent that does not cause a water-absorbing resin to swell. A preferable swelling capacity of a water-absorbing resin during the treatment is less than twice. As the organic solvent, not only a hydrophilic organic solvent but also a hydrophobic organic solvent can be used. Specific examples of the hydrophilic organic solvent encompass: lower alcohols such as methyl alcohol, ethyl alcohol, n-propyl alcohol, isopropyl alcohol, n-butyl alcohol, isobutyl alcohol, and t-butyl alcohol; ketones such as acetone; ethers such as dioxane and tetrahydrofuran; amides such as N,N-dimethylformamide; sulfoxides such as dimethyl sulfoxide; and the like. Examples of the hydrophobic organic solvent encompass: aliphatic hydrocarbons like n-pentane, n-heptane, n-hexane, n-octane, and the like; alicyclic hydrocarbons like cyclohexane, methylcyclohexane, cyclooctane, decalin, and the like; halogenated hydrocarbons like chlorobenzene, bromobenzene, carbon tetrachloride, 1,2-dichloroethane, and the like; aromatic hydrocarbons like benzene, toluene, xylene, and the like; and the like. Among these organic solvents, methyl alcohol, ethyl alcohol, isopropyl alcohol, n-heptane, n-hexane, cyclohexane are preferably used.

In an embodiment of the present invention, in a case where a water-absorbing resin is subjected to hydrophilization treatment with an organic solvent, bringing the organic solvent in a heated state into contact with the water-absorbing resin is preferable because the absorption capacity under pressure and/or the re-wet may be further improved. A heating temperature is preferably equal to or lower than a boiling point of the organic solvent, and is generally about 40°C to 120°C, depending on the type of the organic solvent used. The hydrophilization treatment is preferably carried out in a state in which a water-absorbing resin is dry. In a case where the hydrophilization treatment is carried out in a state in which a water-absorbing resin is moistened with an organic solvent and/or water, caution must be exercised. This is because a decrease in water absorption speed and/or a sharp decrease in water absorption capacity may be caused in a case where such a water-absorbing resin is subjected to crosslinking treatment at and near the surface thereof. Therefore, it is preferable to carry out the hydrophilization treatment in a state in which a solvent for polymerization and moisture are removed through filtering and drying of the water-absorbing resin obtained by reversed phase suspension polymerization or aqueous solution polymerization.

[3-8. Surface-crosslinking step]

The water-absorbing resin (for example, water-absorbing resin powder) obtained through the drying step (and the subsequent optional step) is preferably surface-crosslinked with a surface-crosslinking agent. This surface crosslinking is a process of providing a portion having a high crosslinking density on a surface layer (a portion within several tens of micrometers from the surface of the water-absorbing resin (for example, water-absorbing resin powder)) of the water-absorbing resin (for example, water-absorbing resin powder). Various water absorption properties can be improved by carrying out surface-crosslinking treatment. Note that, in an embodiment of the present invention, a known surface-crosslinking technique is employed as appropriate. Note that the surface-crosslinking agent used in the surface-crosslinking step is also presented as a "postcrosslinking agent" in the known technique in order to be distinguished from the internal crosslinking agent used in the aqueous monomer solution preparation step.

In an embodiment of the present invention, the surface-crosslinking step may be carried out after the above-described drying step or during the drying step. In a known surface-crosslinking step, a surface-crosslinking agent is generally mixed with a hydrogel crosslinked polymer or a crosslinked polymer which is a dried product thereof, and the mixture is heated to carry out a crosslinking reaction. In an embodiment of the present invention, these steps may be separately provided after the above-described drying step, or a surface-crosslinking agent may be added in the drying step to simultaneously carry out surface-crosslinking reaction and drying. Further, in a case where a water-absorbing resin is produced by a batch-type reversed phase suspension polymerization method, the solvent and the hydrogel polymer can be separated by carrying out distillation in the separation step after the polymerization reaction. By adding a surface-crosslinking agent even in the middle of the separation step, a surface-crosslinked water-absorbing resin (for example, water-absorbing resin particles) can be obtained.

[3-9. Other steps]

A method for producing a water-absorbing resin in accordance with an embodiment of the present invention can include, in addition to the above-described steps, optionally a cooling step, a pulverizing step, a hydration (re-moistening) step, a classification step, an other additive addition step, a sizing step, and a fine powder reuse step. Further, the method may further include a conveying step, a storing step, a packing step, a reserving step, and the like.

(Cooling step)

In the cooling step carried out optionally, the particulate dried polymer obtained in the drying step is cooled with use of a known cooling means, so that a particulate dried polymer cooled to a desired temperature can be obtained.

(Pulverizing step)

It is preferable to include the pulverizing step of pulverizing a particulate dried polymer obtained in the drying step (and the subsequent optional cooling step). Carrying out the pulverization step makes it possible to obtain water-absorbing resin powder having a controlled particle diameter or a controlled particle size distribution.

In the pulverizing step, for example, a high-speed rotation pulverizer such as a roll mill, a hammer mill, a screw mill, or a pin mill; a vibration mill; a knuckle-type pulverizer; a cylindrical mixer; or the like is selected as appropriate and used as a pulverizing means.

(Re-moistening step)

The re-moistening step carried out optionally is step of adding, to the water-absorbing resin (for example, water-absorbing resin particles) obtained in the surface-crosslinking step, at least one additive selected from the group consisting of a cationic polymer, a chelating agent, an inorganic reducing agent, $\alpha$-hydroxycarboxylic acid compound, and a polyvalent metal salt. The polyvalent metal salt is preferably a water-soluble polyvalent metal salt and more preferably a water-soluble aluminum salt. Examples of the water-soluble aluminum salt encompass aluminum sulfate.

In the re-moistening step, the above-described additive in the form of an aqueous solution or a dispersion (slurry) is preferably added to a water-absorbing resin (for example, water-absorbing resin particles). Note that the additive may be added, to the water-absorbing resin, together with the above-mentioned surface-crosslinking agent solution, and mixed with the water-absorbing resin. Specifically, the re-moistening step is exemplified by a method described in "(2-7) Re-moistening step" of International Patent Publication No. WO 2015/053372, and such a method can also be employed in an embodiment of the present invention.

(Other additive addition step)

In an embodiment of the present invention, an additive other than the above-described additives can be added in order to give various functions to the water-absorbing resin. Specifically, examples of such an additive encompass a surfactant, a compound having a phosphorus atom, an oxidizer, an organic reducing agent, an inorganic reducing agent, water-insoluble inorganic fine particles, a chelating agent, a polyvalent metal salt, organic powder such as metallic soap, a deodorizing agent, an antibacterial agent, pulp, thermoplastic fibers, and the like. Note that the water-insoluble inorganic fine particles are exemplified by a compound disclosed in "<5> Water-insoluble inorganic fine particles" of International Publication No. WO 2011/040530, and such a compound can be employed in an embodiment of the present invention. Among these additives, water-insoluble inorganic fine particles, hydrophilic fine particles in particular, for example, silica

(silicon dioxide), are preferably added because a resulting water-absorbing resin (for example, water-absorbing resin particles) has an improved compatibility with a liquid, and the water-absorbing resin, when used in an absorbent article, can absorb a water-based liquid in a short time.

(Sizing step)

The "sizing step" means a step of adjusting the particle diameter by disintegrating a water-absorbing resin having been loosely aggregated through the surface-crosslinking step. Note that this "sizing step" encompasses a fine powder removal step and a classification step, which are steps subsequent to the surface-crosslinking step. The sizing step is preferably carried out from the viewpoint of adjusting the particle diameter of a water-absorbing resin and obtaining stable water absorption physical properties.

(Fine powder reuse step)

The "fine powder reuse step" means a step of supplying, to any of the steps, the fine powder generated by, for example, sieve classification in each of the above steps as it is or after having granulated the fine powder. The fine powder reuse step is preferably carried out from the viewpoint of reducing a production loss of a water-absorbing resin.

[4. Application of water-absorbing resin]

An application of the water-absorbing resin in accordance with an embodiment of the present invention is not particularly limited. A preferable application thereof is exemplified by an absorbent body in an absorbent article such as disposable diapers (for infants and for adults), sanitary napkins, and incontinence pads. The water-absorbing resin in accordance with an embodiment of the present invention can be used as an absorbent body in high-concentration disposable diapers in particular.

Further, as a material for the absorbent body, an absorbent material such as pulp fibers can be used together with the water-absorbing resin. In such a case, the content (core concentration) of the water-absorbing resin in the absorbent body is preferably 30 mass% to 100 mass%, more preferably 40 mass% to 100 mass%, even more preferably 50 mass% to 100 mass%, further more preferably 60 mass% to 100 mass%, particularly preferably 70 mass% to 100 mass%, and most preferably 75 mass% to 95 mass%.

In a case where the absorbent body having the core concentration in the above range is used as an upper layer part of an absorbent article, the absorbent article can maintain cleanness, i.e., a state of being white. Further, such an absorbent article is excellent in diffusion property with respect to a body fluid or the like such as urine and/or blood and thus achieves efficient liquid distribution. Therefore, improvement in absorption amount can be expected.

More specifically, the above-described absorbent body may be an absorbent body which contains a water-absorbing resin in accordance with an embodiment of the present invention and (i) which does not contain hydrophilic fibers or (ii) in which the mass of the water-absorbing resin accounts for 50 mass% or more of a total mass of the water-absorbing resin and the hydrophilic fibers.

The hydrophilic fibers are not particularly limited, and examples of the hydrophilic fibers encompass pulp fibers, cotton linter crosslinked cellulose fibers, rayon, cotton, wool, acetate, vinylon, and the like. Further, substances obtained by air-laying these fibers are preferable.

The mass of the water-absorbing resin accounts for 50 mass% or more of the total mass of the water-absorbing resin and the hydrophilic fibers. More specifically, the mass of the water-absorbing resin accounts for 60 mass% or more, 70 mass% or more, 80 mass% or more, 90 mass% or more, or 100 mass% of the total mass of the water-absorbing resin and the hydrophilic fibers.

An embodiment of the present invention can also be configured as follows.

<1> A water-absorbing resin in accordance with an embodiment of the present invention is a water-absorbing resin, wherein the water-absorbing resin is a polyacrylic acid (salt)-based water-absorbing resin, and the water-absorbing resin has: a free swell rate (A) with respect to an aqueous polyethylene oxide solution of 40°C of 0.15 $g \cdot g^{-1} \cdot s^{-1}$ or more; a free swell rate (B) with respect to a physiological saline of 40°C of 0.40 $g \cdot g^{-1} \cdot s^{-1}$ or more; and a free swell rate (A)/free swell rate (B) of 0.20 or more.

<2> A water-absorbing resin in accordance with an embodiment of the present invention is a water-absorbing resin according to <1>, which has a CRC of 25 g/g to 50 g/g.

<3> A water-absorbing resin in accordance with an embodiment of the present invention is a water-absorbing resin according to <1> or <2>, which is an aggregate-like particle of spherical particles.

<4> A water-absorbing resin in accordance with an embodiment of the present invention is a water-absorbing resin

according to any of < 1> to <3>, which has a bulk density of 0.40 g/cm$^3$ to 0.80 g/cm$^3$.

<5> A water-absorbing resin in accordance with an embodiment of the present invention is a water-absorbing resin according to any of <1> to <4>, wherein the free swell rate (A) with respect to the aqueous polyethylene oxide solution of 40°C is 0.25 $g \cdot g^{-1} \cdot s^{-1}$ or more.

<6> A water-absorbing resin in accordance with an embodiment of the present invention is a water-absorbing resin according to any of <1> to <5>, which is obtained by carrying out reversed phase suspension polymerization in a hydrophobic organic solvent.

<7> A water-absorbing resin in accordance with an embodiment of the present invention is a water-absorbing resin according to any of <1> to <6>, which is obtained by extruding spherical particles using an extruder having a porous plate.

<8> An absorbent body in accordance with an embodiment of the present invention is an absorbent body, wherein the absorbent body contains a water-absorbing resin according to any of <1> to <7>, and (i) does not contain hydrophilic fibers or (ii) is such that a mass of the water-absorbing resin accounts for 50 mass% or more of a total mass of the water-absorbing resin and the hydrophilic fibers.

## Examples

The following description will discuss an embodiment of the present invention more concretely with reference to Examples and Comparative Examples below. Note, however, that the present invention is not limited to these Examples and Comparative Examples, and that any Example derived from a proper combination of technical means disclosed in respective different Examples is also encompassed in the scope of the present invention.

Note that electric devices/apparatuses (including devices/apparatuses used to measure physical properties of water-absorbing resins) used in Examples and Comparative Examples each used a 200-V or 100-V electric power supply with a frequency of 60 Hz, unless otherwise specified. Further, the physical properties of water-absorbing resins in Examples of the present invention and Comparative Examples were measured at room temperature (20°C to 25°C) and at a relative humidity of 50% RH, unless otherwise specified.

For convenience, "liter" may be denoted as "1" or "L", and "weight%" may be denoted as "wt%". Furthermore, in the measurement of a trace component, a value equal to or less than a detection limit is denoted as "N.D" (Non Detected).

### [Measurement of physical properties of water-absorbing resin]

### "CRC"

The centrifuge retention capacity (CRC) was measured in conformity with an EDANA method (ERT 441.2-02). Specifically, a water absorption capacity (unit: g/g) was determined after 0.2 g of water-absorbing resin contained in a nonwoven fabric bag was immersed in a large excess of a 0.9 mass% aqueous sodium chloride solution for 30 minutes so as to be allowed to freely swell, and then the water-absorbing resin was drained for 3 minutes with use of a centrifuge (250 G).

### "Ext"

The Ext (water-soluble component) of the water-absorbing resins in Examples of the present invention and Comparative Examples was measured in conformity with an EDANA method (ERT 470.2-02).

### "Ext (1 hr)"

The Ext (1 hr) of the water-absorbing resins in Examples of the present invention and Comparative Examples was in conformity with an EDANA method (ERT 470.2-02). Note that the stirring time was changed to 1 hour.

### "AAP"

The AAP (absorption capacity under pressure) was measured in conformity with an EDANA method (ERT 442.2-02).

### "Moisture content"

The moisture content was measured in conformity with an EDANA method (ERT 430.2-02). Note that, at the time of the measurement, the mass of a sample (water-absorbing resin) was changed to 1.0 g, the drying temperature

was changed to 180°C, and the drying time was changed to 3 hours. Specifically, 1.0 g of the water-absorbing resin was put into an aluminum cup having a 50-mm-diameter bottom surface, and then a total mass W1 (g) of the sample (the water-absorbing resin and the aluminum cup) was weighed accurately. Next, the sample (the water-absorbing resin and the aluminum cup) was allowed to stand still in an oven set to an atmospheric temperature of 180°C. After a lapse of 3 hours, the sample was taken out from the oven, and a total mass W2 (g) was weighed accurately. When the mass of the sample (water-absorbing resin) used in this measurement was assumed to be M (1.0 g), the moisture content $\alpha$ (mass%) of the sample was determined in accordance with the following (Formula 1):

$$\text{Moisture content } \alpha \text{ (mass\%)} = \{(W1 - W2)/M\} \times 100 \quad \text{Formula (1).}$$

"Mass average particle diameter (D50)"

The mass average particle diameter (D50) was measured in accordance with a method described in "(3) Mass-Average Particle Diameter (D50) and Logarithmic Standard Deviation ($\sigma\zeta$) of Particle Diameter Distribution" described in Columns 27 and 28 of US Patent No. 7,638,570. Specifically, 10.0 g of a water-absorbing resin was placed, at a room temperature (20°C to 25°C) and a humidity of 50 RH%, in JIS standard sieves (THE IIDA TESTING SIEVE, diameter: 8 cm) having respective mesh sizes of 850 pm, 710 pm, 600 pm, 500 pm, 425 pm, 300 pm, 212 pm, 150 pm, and 45 pm, and a vibration classifier (IIDA SIEVE SHAKER, Type: ES-65, Ser. No. 0501) was used to carry out classification for 5 minutes. After that, a residual percentage R on each of the sieves was plotted on a logarithmic probability paper. Thus, a particle size corresponding to R=50 mass% was read as the mass average particle diameter (D50).

"Free swell rate (A) with respect to 3 weight% aqueous polyethylene oxide solution of 40°C"

An aqueous polyethylene oxide solution having a concentration of 3 weight%, a liquid temperature of 40°C, and a viscosity of 10±1 mPa·s under those conditions was prepared with use of polyethylene oxide (PEO-1, viscosity average molecular weight of 150,000 to 400,000, available from Sumitomo Seika Chemicals Co., Ltd.). Note that a vibration viscometer (model: VM-10A) available from SEKONIC CORPORATION was used to measure the viscosity. In the following, 25-ml and 50-ml glass beakers were used after being kept warm at 40°C.

0.50 g of water-absorbing resin was placed in the 25-ml glass beaker (inner diameter of 32 mm to 34 mm, height of 50 mm), and the weight was measured (W3). At this time, the upper surface of the water-absorbing resin placed in the beaker was made level. If necessary, the upper surface of the water-absorbing resin was leveled by carefully hitting the beaker or by taking other measure.

In the center of the beaker containing the water-absorbing resin, a glass funnel capable of pouring a 3 weight% aqueous polyethylene oxide solution at a flow velocity of 5 g/sec to 6 g/sec was set such that the tip of the funnel was positioned at a height of 50 mm from the bottom of the beaker. Next, 10 g of the 3 weight% aqueous polyethylene oxide solution adjusted to 40°C±0.5°C was weighed in the 50-ml glass beaker and poured gently and quickly into the funnel.

Time measurement was started at the same time when the 3 weight% aqueous polyethylene oxide solution poured into the funnel came into contact with the water-absorbing resin. After the 3 weight% aqueous polyethylene oxide solution had been poured into the funnel, the funnel was removed at the point in time when no water droplets had fallen from the funnel for 5 seconds. Immediately after the start of the time measurement, the upper surface of the water-absorbing resin was present below a liquid level of the 3 weight% aqueous polyethylene oxide solution in the beaker.

Then, when the liquid level of the 3 weight% aqueous polyethylene oxide solution in the beaker into which the 3 weight% aqueous polyethylene oxide solution had been poured was visually observed at an angle of approximately 20°, the time measurement was ended (unit: seconds) (tS1) at the point in time when the liquid level was replaced with the upper surface of the water-absorbing resin having absorbed (at the point in time when the shape of gel particles on the upper surface of the water-absorbing resin could be recognized, and no liquid remained in the center of the upper surface of the water-absorbing resin).

Next, the weight (unit: g) of the 25-ml glass beaker after the pouring of the 3 weight% aqueous polyethylene oxide solution was measured (W4).

The weight (W5, unit: g) of the 3 weight% aqueous polyethylene oxide solution having been poured into the 25-ml glass beaker was determined by the following formula (a). The free swell rate (A) was calculated by the following formula (b).

$$\text{Formula (a): } W5 \text{ (g)} = W4 \text{ (g)} - W3 \text{ (g)}$$

$$\text{Formula (b): free swell rate (A) } [g \cdot g^{-1} \cdot s^{-1}] = W5/(tS1 \times \text{mass (g) of water-absorbing resin})$$

"Free swell rate (B) with respect to 0.9 mass% aqueous sodium chloride solution"

The viscosity of a 0.9 mass% aqueous sodium chloride solution of 40°C was measured with use of a vibration viscometer (model: VM-10A) available from SEKONIC CORPORATION and found to be 0 mPa·s to 1 mPa·s. In the following, 25-ml and 50-ml glass beakers were used after being kept warm at 40°C.

1.00 g of water-absorbing resin was placed in the 25-ml glass beaker (inner diameter of 32 mm to 34 mm, height of 50 mm). At this time, the upper surface of the water-absorbing resin placed in the beaker was made level. If necessary, the upper surface of the water-absorbing resin was leveled by carefully hitting the beaker or by taking other measure.

Next, 20 g of the 0.9 mass% aqueous sodium chloride solution adjusted to 40°C±0.5°C was weighed in the 50-ml glass beaker, and a total weight (unit: g) of the 0.9 mass% aqueous sodium chloride solution and the glass beaker was measured (W6). The whole amount of the weighed 0.9 mass% aqueous sodium chloride solution was gently and quickly poured into the 25-ml beaker containing the water-absorbing resin.

Time measurement was started at the same time when the 0.9 mass% aqueous sodium chloride solution poured into the beaker came into contact with the water-absorbing resin. Immediately after the start of the time measurement, the upper surface of the water-absorbing resin was present below a liquid level of the 0.9 mass% aqueous sodium chloride solution in the beaker. Then, when the liquid level of the 0.9 mass% aqueous sodium chloride solution in the beaker into which the 0.9 mass% aqueous sodium chloride solution had been poured was visually observed at an angle of approximately 20°, the time measurement was ended (unit: seconds) (tS2) at the point in time when the liquid level was replaced with the upper surface of the water-absorbing resin having absorbed (at the point in time when the shape of gel particles on the upper surface of the water-absorbing resin could be recognized, and no liquid remained in the center of the upper surface of the water-absorbing resin).

Next, the weight (unit: g) of the 50-ml glass beaker after the pouring of the 0.9 mass% aqueous sodium chloride solution was measured (W7).

The weight (W8, unit: g) of the 0.9 mass% aqueous sodium chloride solution having been poured into the 25-ml glass beaker was determined by the following formula (c).

The free swell rate (B) was calculated by the following formula (d).

$$\text{Formula (c): } W8 \text{ (g)} = W6 \text{ (g)} - W7 \text{ (g)}$$

$$\text{Formula (d): free swell rate (B) } [g \cdot g^{-1} \cdot s^{-1}] = W8/(tS2 \times \text{mass (g) of water-absorbing resin})$$

"Free swell rate ratio"

The free swell rate ratio was calculated in accordance with the following formula:

$$\text{Free swell rate ratio} = \text{free swell rate (A) } [g \cdot g^{-1} \cdot s^{-1}]/\text{free swell rate (B) } [g \cdot g^{-1} \cdot s^{-1}]$$

"Bulk density"

The bulk density of a water-absorbing resin was measured in conformity with an EDANA method (ERT 460.2-02).

"Number average particle diameter"

A scanning electron microscope photograph (SEM) of a water-absorbing resin or water-absorbing resin powder was taken. From the photograph, 50 primary particles on the front of the aggregate-like particle were randomly selected.

The major axis and minor axis of each primary particle were measured, and an average value of the measured values was assumed to be a primary particle diameter. An average value of the primary particle diameters was calculated, and the average value was assumed to be an average primary particle diameter of the water-absorbing resin.

"Viscosity"

The viscosity was measured with use of a vibration viscometer (model: VM-10A) available from SEKONIC CORPORATION. Specifically, 40 g to 45 g of a test liquid was charged into a screw tube (No. 7, 50 ml, code 730-09) available from Maruemu Corporation, the liquid temperature was adjusted to 40°C, the level of the liquid was adjusted so that a rod part on the top of the detector was immersed by 2 mm to 3 mm in the test liquid, and the viscosity (mPa·s) was measured.

"Surface tension"

The surface tension, which is a surface tension of an aqueous solution obtained by dispersing a water-absorbing resin in a 0.9 mass% aqueous sodium chloride solution, was measured by a method described in WO 2015/129917.

[Example 1]

A hydrogel polymer was prepared in accordance with a production process illustrated in Fig. 1 of International Publication No. WO 2020/067310.

Used as a dispersion apparatus was a two-fluid spray nozzle (external mixing type, spray nozzle inner diameter: 0.5 mm, type: SETO07507S303+TS303, available from H. IKEUCHI &, Co., Ltd.) illustrated in Fig. 9 of International Publication No. WO 2020/067310. Used as a reaction apparatus was a PFA tube (internal diameter: 25 mm, total length: 10 m) placed vertically.

As a stage preparatory to polymerization reaction, a solution obtained by adding, to n-heptane (density: 0.68 g/ml) as a hydrophobic organic solvent, a 0.005 mass% sucrose fatty acid ester (trade name: DK Ester (registered trademark) F-50, available from DKS Co., Ltd., HLB=6) was charged into an auxiliary fluid flow channel (second supply tube) of the two-fluid spray nozzle, the reaction apparatus, and the separation apparatus, and into pipes connecting the flow channel and the apparatuses. The position of the two-fluid spray nozzle was adjusted so that a tip of the two-fluid spray nozzle was immersed in a continuous phase composed of a hydrophobic organic solvent contained in the reaction apparatus.

Subsequently, a liquid feed pump was operated, and the circulation of the organic solvent was started at a flow rate of 1000 ml/min. In this production method, the path of the circulated organic solvent was branched into a path for charging into the reaction apparatus via the two-fluid spray nozzle and a path for directly charging into the reaction apparatus. The flow rate of the organic solvent charged into the reaction apparatus via the two-fluid spray nozzle was set to 800 ml/min, and the flow rate of the organic solvent directly charged into the reaction apparatus was set to 200 ml/min. Further, the flow velocity of the organic solvent at the tip portion of the two-fluid spray nozzle was 7.86 m/sec. Further, a heat exchanger was operated to heat the circulated organic solvent so that the set temperature became 85°C.

Next, acrylic acid, a 48.5 mass% aqueous sodium hydroxide solution, and ion-exchanged water were mixed, and, further, N,N'-methylenebisacrylamide and trisodium diethylenetriamine pentaacetate were blended to prepare a monomer solution (1). Further, separately, sodium persulfate and ion-exchanged water were mixed to prepare a 10 mass% aqueous sodium persulfate solution (1).

Subsequently, the monomer solution (1) and the aqueous sodium persulfate solution (1) both of which had been obtained by the above-described operation were separately supplied to a mixing apparatus and mixed to prepare an aqueous monomer solution (1). The monomer concentration of the aqueous monomer solution (1) was 43 mass%, and the neutralization rate was 70 mol%. Further, N,N'-methylenebisacrylamide which served as an internal crosslinking agent was 0.015 mol% with respect to the monomer, trisodium diethylenetriamine pentaacetate which served as a chelating agent was 100 ppm with respect to the monomer, and sodium persulfate which served as a polymerization initiator was 0.1 g/mol with respect to the monomer.

Next, the aqueous monomer solution (1) prepared by the mixing apparatus was promptly fed to the aqueous monomer solution flow channel (first supply tube) of the two-fluid spray nozzle. After that, with use of the two-fluid spray nozzle, the aqueous monomer solution (1) was supplied, together with the organic solvent, to the reaction apparatus at a flow rate of 40 ml/min (23.6 g/min). The aqueous monomer solution (1) was supplied so as to be in the same direction (parallel flow) as a circulation direction of the organic solvent forming the continuous phase. Note that the flow velocity of the aqueous monomer solution (1) at the tip portion of the two-fluid spray nozzle was 0.85 m/sec. Further, the liquid temperature of the aqueous monomer solution (1) before being supplied to the two-fluid spray nozzle was maintained at 25°C.

The aqueous monomer solution (1) supplied by the two-fluid spray nozzle was dispersed in the form of droplets in the continuous phase. The ratio (W/O ratio) of the aqueous monomer solution (1) to the organic solvent forming the continuous phase was 3.3 volume%.

Then, the dispersion obtained as described above was supplied to the reaction apparatus. The droplets composed of the aqueous monomer solution (1) were polymerized while falling in the reaction apparatus filled with a hydrophobic organic solvent which served as the continuous phase, and a microspherical hydrogel polymer (1) was found in the vicinity of a discharge port of the reaction apparatus.

The hydrogel polymer (1) obtained by the series of operations was continuously supplied, together with the hydrophobic organic solvent, from the reaction apparatus to the separation apparatus through a joining part, and the hydrogel polymer (1) was separated from the organic solvent in the separation apparatus. In the separation apparatus, the hydrogel polymer (1) was an aggregate of microspherical particles, and the size of the aggregate was 5 mm to 10 mm.

The hydrogel polymer (1) (gel temperature: 90°C) was charged into an extruder-type gel sizing apparatus having a screw and a porous plate having a hole diameter of 0.8 mm, and the hydrogel polymer (1) was discharged from the gel sizing apparatus to obtain a sized gel (1).

Subsequently, by allowing hot air of 105°C to pass through for 45 minutes, the particulate hydrogel polymer (1) was dried to obtain a particulate dried polymer (1).

Subsequently, the dried polymer (1) was supplied to a roll mill (WML type roll pulverizer available from Inokuchi Giken Ltd.) and pulverized to adjust the particle size, and further classified with use of a sieve having a mesh size particle diameter of 150 pm to obtain water-absorbing resin powder (1).

To 1000 ml of methanol heated to 60°C, 20 g of the water-absorbing resin powder (1) was added and stirred for 1 hour, followed by filtration and drying to carry out hydrophilization treatment.

To 100 parts by mass of the water-absorbing resin powder (1), a surface-crosslinking agent solution composed of 0.015 parts by mass of ethylene glycol diglycidyl ether, 1.0 part by mass of propylene glycol, and 3.0 parts by mass of ion-exchanged water was sprayed, followed by uniform mixing with use of a high speed continuous mixer.

A resulting mixture was introduced into a heat treatment machine adjusted to an atmospheric temperature of 195°C±2°C and heat-treated for 40 minutes. Then, the powder temperature was forcibly cooled to 60°C to obtain surface-crosslinked water-absorbing resin powder (1). Hereinafter, the surface-crosslinked water-absorbing resin powder is referred to as "water-absorbing resin particles".

To 100 parts by mass of the water-absorbing resin particles (1), a mixture solution composed of 0.40 parts by mass of a 27.5 weight% aqueous aluminum sulfate solution (8 weight% in terms of aluminum oxide), 0.134 parts by mass of a 60 mass% aqueous sodium lactate solution, and 0.002 parts by mass of propylene glycol was added. After the addition, a resulting mixture was dried at 60°C for 30 minutes under windless conditions, water was added so that the water-absorbing resin particles (1) had a moisture content of 10 mass%, and sizing was carried out by passage through a JIS standard sieve having a mesh size of 1000 pm to obtain a water-absorbing resin (1). Table 1 shows various physical properties of the obtained water-absorbing resin (1).

[Example 2]

Operations were carried out in the same manner as in Example 1 to obtain a water-absorbing resin (2), except that, in Example 1, (i) a change was made from the addition of the 0.005 mass% sucrose fatty acid ester (trade name: DK Ester (registered trademark) F-50, available from DKS Co., Ltd., HLB=6) which served as a dispersing aid to the addition of a 0.005 mass% maleic anhydride-modified ethylene-propylene copolymer (trade name: Hi-WAX (registered trademark) 1105A, available from Mitsui Chemicals Co., Ltd.), and (ii) the internal crosslinking agent was changed from N,N'-methylenebisacrylamide which was 0.015 mol% with respect to the monomer to polyethyleneglycol diacrylate (average polymerization degree: 9) which was 0.008 mol% with respect to the monomer. Table 1 shows various physical properties of the obtained water-absorbing resin (2).

[Comparative Example 1]

500 ml of n-heptane was added to a 1000-ml capacity five-necked cylindrical round-bottomed flask equipped with a stirrer, a reflux condenser, a dropping funnel, a thermometer, and a nitrogen gas introduction tube. To this, 0.92 g of a sucrose fatty acid ester having an HLB of 3.0 (surfactant: S-370 available from Mitsubishi Chemical Corporation) was added and dispersed, and the temperature was increased to dissolve the surfactant, followed by cooling to 55°C.

Separately from the above, 92 g of an 80 weight% aqueous acrylic acid solution was added to a 500-ml capacity Erlenmeyer flask. To this, while being cooled from the outside, 102.2 g of a 30 weight% aqueous sodium hydroxide solution was added dropwise to neutralize 75 mol% of acrylic acid, so that an aqueous solution of partially neutralized acrylic acid was prepared. Further, 50.2 g of water, 0.11 g of potassium persulfate which served as a polymerization initiator, and 9.2 mg of ethylene glycol diglycidyl ether which served as a crosslinking agent were added to prepare an

aqueous monomer solution for first-stage polymerization.

The whole amount of this aqueous monomer solution for first-stage polymerization was added for dispersion to the above-described five-necked cylindrical round-bottomed flask while being stirred by the stirrer at a rotation speed of 500 rpm, the inside of the system was sufficiently replaced with nitrogen, and then the temperature was increased. The bath temperature was maintained at 70°C, the polymerization reaction was carried out for 1 hour, and then a polymerization slurry liquid was cooled to room temperature.

To another 500-ml capacity Erlenmeyer flask, 119.1 g of an 80 weight% aqueous acrylic acid solution was added, and 132.2 g of a 30 weight% aqueous sodium hydroxide solution was added dropwise while being cooled to neutralize 75 mol% of acrylic acid. Further, 27.4 g of water, 0.14 g of potassium persulfate, and 35.7 mg of ethylene glycol diglycidyl ether were added to prepare an aqueous monomer solution for second-stage polymerization, and the aqueous monomer solution for second-stage polymerization was cooled in an ice water bath.

While being stirred by the stirrer at a rotation speed of 1000 rpm, the whole amount of this aqueous monomer solution for second-stage polymerization was added to the polymerization slurry liquid, the inside of the system was sufficiently replaced with nitrogen again, and then the temperature was increased. The bath temperature was maintained at 70°C, and the second-stage polymerization reaction was carried out for 2 hours. After completion of the polymerization, 0.53 g of a 40 weight% aqueous pentasodium diethylenetriamine pentaacetate solution as an aminocarboxylic acid-based metal chelating agent was added to the hydrogel-like substance dispersed in n-heptane while being stirred. After that, 266 g of water was extracted from the hydrogel-like substance to the outside of the system by azeotropic dehydration. To the obtained gel-like substance, 8.44 g of a 2 weight% aqueous ethylene glycol diglycidyl ether solution was added. Further, water and n-heptane were removed by distillation, followed by drying to obtain a dried polymer. This dried polymer was passed through a sieve with a mesh size of 850 pm, and 0.1 mass% amorphous silica (available from Oriental Silicas Corporation, TOKUSIL (registered trademark) NP-S) based on the mass of the dried polymer was mixed with the dried polymer to obtain a comparative water-absorbing resin (1) containing amorphous silica. Table 1 shows various physical properties of the obtained comparative water-absorbing resin (1).

[Comparative Example 2]

An aqueous monomer solution was prepared by mixing 67.0 parts of a 37% aqueous sodium acrylate solution, 10.2 parts of acrylic acid, 0.079 parts of polyethyleneglycol diacrylate (average number of ethylene oxide units: 8), and 22.0 parts of water. Nitrogen was blown into the aqueous monomer solution in a vat to reduce the dissolved oxygen in the solution to 0.1 ppm or less.

Subsequently, the temperature of the aqueous monomer solution was adjusted to 18°C in a nitrogen atmosphere, and then 0.16 parts of a 5% aqueous sodium persulfate solution, 0.16 parts of a 5% aqueous 2,2'-azobis(2-amidinopropane) dihydrochloride solution, 0.15 parts of a 0.5% aqueous L-ascorbic acid solution, and 0.17 parts of a 0.35% aqueous hydrogen peroxide solution were sequentially added dropwise while being stirred. Polymerization started immediately after the dropwise addition of hydrogen peroxide. After that, stirring was stopped, and after 10 minutes, the temperature of the monomer reached the peak temperature. The peak temperature was 85°C. Subsequently, the vat was immersed in a hot water bath of 80°C, followed by aging for 10 minutes. A resulting clear hydrogel was crushed with a meat chopper and then dried at 180°C for 30 minutes.

A dried product was pulverized with a pulverizer, passed through a 500-pm sieve, and classified into particles remaining on a 105-pm sieve to obtain water-absorbing resin powder. A composition liquid composed of 0.002 parts of diethylenetriamine pentaacetic acid, 0.05 parts of ethylene glycol diglycidyl ether, 1 part of propylene glycol, 3 parts of water, and 1 part of isopropyl alcohol was mixed with 100 parts of the water-absorbing resin powder, and a resulting mixture was heat-treated at 180°C for 40 minutes to obtain comparative water-absorbing resin particles (2).

Subsequently, 0.3 mass% of silicon dioxide (available from Nippon Aerosil Co., Ltd.; Aerosil (registered trademark) 200) as inorganic powder was added to 100 parts of the comparative water-absorbing resin particles (2) to obtain a comparative water-absorbing resin (2). Table 1 shows various measurement results of physical properties of this comparative water-absorbing resin (2).

[Comparative Example 3]

A comparative water-absorbing resin (3) was obtained in conformity with Example 2 of Japanese Patent Application Publication, Tokukai, No. 2006-068731. Table 1 shows various physical properties of the obtained comparative water-absorbing resin (3).

[Table 1]

| | CRC [g/g] | Free swell rate (A) [$g \cdot g^{-1} \cdot s^{-1}$] | Free swell rate (B) [$g \cdot g^{-1} \cdot s^{-1}$] | Free swell rate ratio | Bulk density [g/ml] | Number average particle diameter of primary particle diameter [μm] | AAP 2.06 kPa [g/g] | Ext [%] | Ext (1Hr) [%] | D50 [μm] | Particles of less than 150 μm [mass%] | Surface tension [mN/m] |
|---|---|---|---|---|---|---|---|---|---|---|---|---|
| Example 1 | 32 | 0.27 | 0.69 | 0.38 | 0.71 | 40 | 29 | 24 | 5 | 380 | 1 | 72 |
| Example 2 | 39 | 0.18 | 0.63 | 0.28 | 0.70 | 85 | 28 | 27 | 10 | 360 | 1 | 71 |
| Comparative Example 1 | 33 | 0.14 | 0.52 | 0.27 | 0.70 | 60 | 33 | 22 | 4 | 350 | 1 | 62 |
| Comparative Example 2 | 34 | 0.47 | 0.36 | 1.32 | 0.64 | - | 28 | 16 | 8 | 380 | 3 | 72 |
| Comparative Example 3 | 34 | 0.24 | 1.9 | 0.13 | 0.69 | 80 | 31 | 24 | 10 | 370 | 1 | 67 |

From Table 1, it can be seen that the free swell rate (A) and the free swell rate (B) are compatible with each other in Examples as compared with those in Comparative Examples.

Industrial Applicability

An embodiment of the present invention is applicable to an absorbent body in an absorbent article such as disposable diapers (for infants and for adults), sanitary napkins, and incontinence pads.

## Claims

**1.** A water-absorbing resin, wherein

said water-absorbing resin is a polyacrylic acid (salt)-based water-absorbing resin, and
said water-absorbing resin has:

a free swell rate (A) with respect to an aqueous polyethylene oxide solution of 40°C of 0.15 $g \cdot g^{-1} \cdot s^{-1}$ or more;
a free swell rate (B) with respect to a physiological saline of 40°C of 0.40 $g \cdot g^{-1} \cdot s^{-1}$ or more; and
a free swell rate (A)/free swell rate (B) of 0.20 or more.

**2.** The water-absorbing resin according to claim 1, which has a CRC of 25 g/g to 50 g/g.

**3.** The water-absorbing resin according to claim 1 or 2, which is an aggregate-like particle of spherical particles.

**4.** The water-absorbing resin according to any one of claims 1 to 3, which has a bulk density of 0.40 $g/cm^3$ to 0.80 $g/cm^3$.

**5.** The water-absorbing resin according to any one of claims 1 to 4, wherein the free swell rate (A) with respect to the aqueous polyethylene oxide solution of 40°C is 0.25 $g \cdot g^{-1} \cdot s^{-1}$ or more.

**6.** The water-absorbing resin according to any one of claims 1 to 5, which is obtained by carrying out reversed phase suspension polymerization in a hydrophobic organic solvent.

**7.** The water-absorbing resin according to any one of claims 1 to 6, which is obtained by extruding spherical particles using an extruder having a porous plate.

**8.** An absorbent body, wherein
said absorbent body contains a water-absorbing resin according to any one of claims 1 to 7, and (i) does not contain hydrophilic fibers or (ii) is such that a mass of the water-absorbing resin accounts for 50 mass% or more of a total mass of the water-absorbing resin and the hydrophilic fibers.

**INTERNATIONAL SEARCH REPORT**

International application No.
**PCT/JP2022/019629**

**A. CLASSIFICATION OF SUBJECT MATTER**

***B01J 20/26***(2006.01)i; ***B01J 20/28***(2006.01)i; ***B01J 20/30***(2006.01)i; ***C08F 2/18***(2006.01)i; ***C08F 20/06***(2006.01)i; ***C08J 3/12***(2006.01)i
FI: B01J20/26 D; B01J20/28 Z; B01J20/30; C08F2/18; C08F20/06; C08J3/12 Z CEY

According to International Patent Classification (IPC) or to both national classification and IPC

**B. FIELDS SEARCHED**

Minimum documentation searched (classification system followed by classification symbols)

B01J20/00-20-34, C08F2/00-301/00, A61F13/00-13/84

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/067310 A1 (NIPPON SHOKUBAI CO., LTD.) 02 April 2020 (2020-04-02) claims, paragraphs [0175], [0176] | 1-8 |
| Y | WO 2018/092864 A1 (NIPPON SHOKUBAI CO., LTD.) 24 May 2018 (2018-05-24) claims, paragraphs [0039]-[0042] | 1-8 |
| Y | JP 2005-186015 A (SAN-DIA POLYMER LTD.) 14 July 2005 (2005-07-14) paragraphs [0002]-[0005], [0101] | 1-8 |
| A | JP 2016-506981 A (BASF SE) 07 March 2016 (2016-03-07) | 1-8 |
| A | JP 2003-235889 A (SAN-DIA POLYMER LTD.) 26 August 2003 (2003-08-26) | 1-8 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 July 2022** | **19 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/019629**

| Patent document cited in search report | Publication date (day/month/year) | Patent family member(s) | Publication date (day/month/year) |
|---|---|---|---|
| WO 2020/067310 A1 | 02 April 2020 | CN 112789297 A<br>KR 10-2021-0049869 A | |
| WO 2018/092864 A1 | 24 May 2018 | US 2019/0329219 A1<br>claims, paragraphs [0107]-[0109]<br>CN 109996835 A<br>EP 3543280 A1<br>KR 10-2019-0077541 A | |
| JP 2005-186015 A | 14 July 2005 | (Family: none) | |
| JP 2016-506981 A | 07 March 2016 | US 2015/0376318 A1<br>CN 104936989 A<br>EP 2951212 A1<br>WO 2014/118024 A1 | |
| JP 2003-235889 A | 26 August 2003 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020137241 A
- WO 2002085959 A
- WO 2011023572 A
- WO 2011040530 A
- US 7638570 B
- WO 2009025235 A
- WO 2013018571 A
- WO 2006100300 A
- WO 2011025012 A
- WO 2011025013 A
- WO 2011111657 A
- WO 2015053372 A
- WO 2015129917 A
- WO 2020067310 A
- JP 2006068731 A


### Non-patent literature cited in the description


- **FREDRIC L. BUCHHOLZ ; ANDREW T. GRAHAM.** Modern Superabsorbent Polymer Technology. WILEY-VCH, 1998